# EUROPÄISCHE PATENTSCHRIFT

(11) **EP 3 463 255 B1**
(45) Veröffentlichungstag und Bekanntmachung des Hinweises auf die Patenterteilung: **07.04.2021**
(21) Anmeldenummer: 17727518.7
(22) Anmeldetag: 22.05.2017
(51) Int. Cl.: A61K 6/60, A61K 6/818, C04B 41/00, C04B 41/47, C04B 41/48, C04B 41/82, C04B 41/83, C04B 111/00

(54) **MIT EINER ORGANISCHEN VERBINDUNG GEFÜLLTER KERAMISCHER ROHLING MIT VERBESSERTEN BEARBEITUNGSEIGENSCHAFTEN**
CERAMIC BLANK FILLED WITH AN ORGANIC COMPOUND AND WITH IMPROVED MACHINING PROPERTIES
ÉBAUCHE CÉRAMIQUE CHARGÉE D'UN COMPOSÉ ORGANIQUE PRÉSENTANT DES PROPRIÉTÉS DE FAÇONNAGE AMÉLIORÉES

(30) Priorität: 23.05.2016 DE 102016109437
(43) Veröffentlichungstag der Anmeldung: 10.04.2019
(73) Patentinhaber: Kulzer GmbH, 63450 Hanau (DE)
(72) Erfinder: WOLDEGERGIS, Yohannes, 63457 Hanau (DE); WEITZEL, Tim, 63571 Gelnhausen (DE); HORMANN, Stefan, 63636 Brachttal (DE)
(74) Vertreter: Bendele, Tanja
(86) Internationale Anmeldenummer: PCT/EP2017/062270
(87) Internationale Veröffentlichungsnummer: WO 2017/202770

(56) Entgegenhaltungen:
- EP-A1- 2 233 449
- DE-A1- 4 141 632
- DE-A1-102006 034 551

## Beschreibung

Dentaler keramischer Rohling, insbesondere gefüllter Fräsrohling, umfassend mindestens eine organische Verbindung, wie ein in dem Rohling polymerisiertes Polymer, zur Verbesserung der Bearbeitungseigenschaften des Rohlings, wobei der Rohling ein offenporiges keramisches Gerüst aufweist, das zu 2 bis 50 Gew.-% mindestens eine organische Verbindung in Bezug auf die Gesamtzusammensetzung des dentalen keramischen Rohlings aufweist sowie ein Verfahren zur Herstellung des Rohlings. Der erfindungsgemäße Fräsrohling zeigt deutlich verbesserte Materialeigenschaften gegenüber ungefüllt rein keramischen Fräsrohlingen, die in CAD/CAM-Verfahren zur Rohlingen von prothetischen Formteilen gefräst werden.

CAD/CAM-Verfahren sind computergestützte Herstellverfahren von ästhetischen prothetischen dentalen Versorgungen, wie Brücken und Kronen. Dabei bedeutet in CAD/CAM die englische Abkürzung CAD Computer Aided Design und CAM für Computer Aided Manufacturing.

So werden in automatisierten materialabtragenden Verfahren 3D-Werkstücke aus den CAD-Daten in einem CAM-Verfahren hergestellt. Übliche abtragende Verfahren umfassen Fräsen, Bohren, Schneiden, Abplatzen, Schmelzen und/oder mindestens zwei der abtragenden Verfahrensschritte. Neben den bekannten CAD/CAM-Verfahren kann zukünftig auch das sogenannte Lasermilling, einer mittels Laserstrahlen bewirkten Materialabtragung, zur Bearbeitung von Rohlingen zur Herstellung von prothetischen dentalen Versorgungen verwendet werden. Um die gewünschten Resultate zu erhalten, sind die Materialeigenschaften der Rohlinge spezifisch an den Lasermilling-Prozess anzupassen.

Die Aufgabe der Erfindung bestand darin, einen dentalen keramischen Rohling mit verbesserten Materialeigenschaften zu entwickeln, der eine automatisierte Herstellung von individuellen prothetischen Formteilen aus Rohlingen erlaubt. Vorzugsweise soll auch der Schrumpf des bearbeiteten Rohlings beim Sintern reduziert werden, so dass weitgehend auf die Ausbildung von Tragekonstruktionen, wie Stegen zwischen den gefrästen Rohlingen der prothetischen Formteile und dem Restrohling verzichtet werden kann. Insbesondere sollen die Bearbeitungseigenschaften in Bezug auf die Bruchfestigkeit und/oder den E-Modul verbessert werden.

Gelöst werden die Aufgaben der Erfindung durch den erfindungsgemäßen dentalen keramischen Rohling, insbesondere den gefüllten keramischen Rohling, nach Anspruch 1 sowie durch das Verfahren zur Herstellung des Rohlings nach Anspruch 10. Bevorzugte Ausführungsformen des Rohlings werden in den Unteransprüchen und detaillierter in der Beschreibung dargestellt.

Erfindungsgemäß wird eine offenporige keramische Matrix synonym zu offenporiges keramisches Gerüst eines teilgesinterten Rohlings mit mindestens einer organischen Verbindung infiltriert. Nach der Infiltration und Verfestigung der mindestens einen Verbindung weist das keramische Gerüst verbesserte Materialeigenschaften auf. Die Infiltration des Rohlings, wie des offenporigen keramischen Gerüstes, erfolgt in einem Bad mindestens einer flüssigen oder aus der Gasphase abscheidbaren organischen Verbindung bis zur vollständigen Sättigung des offenporigen keramischen Gerüstes. Die Verfestigung erfolgt bspw. durch Polymerisation eines flüssigen infiltrierten Monomers, einer Monomermischung oder durch Abkühlen eines geschmolzenen Wachses. Nach erfolgter Materialbearbeitung des Rohlings, wie dem Fräsen, wird die organische Verbindung aus dem Gerüst ausgebrannt, d.h. die Verbindung wird pyrolysiert, so dass das offenporige keramische Gerüst wieder freigelegt wird.

Die Verstärkung von Röhlingen durch Infiltration des keramischen Gerüstes mit einem niedrigviskosen flüssigen Kunstharz wird in DE 10 2006 034 551 beschrieben.

Gegenstand der Erfindung ist ein dentaler keramischer Rohling, insbesondere ein teilgesinterter Rohling, wie ein Weißling, umfassend mindestens eine organische Verbindung, wobei der Rohling ein offenporiges keramisches Gerüst aufweist, das 5 bis 50 Gew.-% mindestens einer organischen Verbindung in Bezug auf die Gesamtzusammensetzung des dentalen keramischen Rohlings aufweist, vorzugsweise weist der Rohling 5 bis 25 Gew.-%, weiter bevorzugt 10 bis 25 Gew.-%, besonders bevorzugt 15 bis 25 Gew.-% mindestens einer organischen Verbindung, vorzugsweise HEMA, Polymere von HEMA oder ein dentales Wachs, in Bezug auf die Gesamtzusammensetzung auf. Der erfindungsgemäße Rohling kann auch als mit mindestens einer organischen Verbindung gefüllter Rohling bezeichnet werden.

Das offenporige keramische Gerüst des Rohlings weist vorzugsweise eine offenporige Porosität von 10 bis 80 % auf, insbesondere von 20 bis 70 %, bevorzugt von 30 bis 60%.

Das offenporige keramische Gerüst des Rohlings umfasst vorzugsweise Zirkondioxid, Aluminiumoxid, Mischoxid(e) umfassend Zirkondioxid und Zirkondioxid und/oder Siliciumcarbid. Besonders bevorzugt sind Zirkondioxid, insbesondere mit einem Gehalt an Zirkondioxid von größer gleich 50 Gew.-%, insbesondere mit einem Gehalt größer gleich 70 Gew.-% an Zirkondioxid. Alternativ bevorzugt ist ein Aluminiumoxid mit einem Gehalt von größer gleich 95 Gew.-%, insbesondere größer gleich 99,7 Gew.-%, bevorzugt größer gleich 99,99 Gew.-%. Des Weiteren umfassen besonders bevorzugte keramische Gerüste einen Gehalt an Zirkondioxid von größer gleich 50 bis 100 Gew.-% optional zusätzlich umfassend Magnesium, wie (Mg-PSZ, teilstabilisert), MgO, Zirkondioxid (Y-TZP, teilstabilisert) umfassend Y₂O₃, Zirkondioxid HIP Zustand (Y-TZP, teilstabilisert) umfassend Y₂O₃, ZrO₂/Al₂O₃ Mischoxid, SiSiC ein mit Silicium infiltriertes Siliciumcarbid, Siliciumcarbid gesintert ohne freies Silicium, heißgepresstes Siliciumcarbid ohne freies Silicium. Bevorzugt sind keramische Gerüste mit einem Gehalt an Zirkondioxid, Aluminiumoxid, Mischoxid umfassend Zirkondioxid und Zirkondioxid und/oder Siliciumcarbid von größer gleich 70 Gew.-% bis 100 Gew.-%, insbesondere größer gleich 73 Gew.-%, bevorzugt größer gleich 85 Gew.-%, größer gleich 90 Gew.-%, wobei das Gerüst vorzugsweise mit einer Yttrium-Verbindung und/oder Magnesiumoxid stabilisiert ist.

Gegenstand der Erfindung ist ein keramischer Rohling umfassend ein keramisches Gerüst, insbesondere ein offenporiges keramisches Gerüst, umfassend einen Gehalt an Zirkondioxid von 50 bis 100 Gew.-%, insbesondere von 70 bis 100 Gew.-%, bevorzugt von 85 Gew.-% bis 100 Gew.-%, besonders bevorzugt von 90 bis 100 Gew.-%, und optional umfassend einen Gehalt anderer Metalloxide, Halbmetalloxide, Siliciumcarbid, insbesondere eines der vorgenannten, oder Gemischen dieser ausgewählt aus Yttrium, Aluminium, Magnesium, Kalium, Calcium, Lithium, und optional Silicium, mit einem Gehalt von 0 bis 50 Gew.-%, insbesondere von 0 bis 30 Gew.-%, bevorzugt von 0 bis 15 Gew.-%, besonders bevorzugt von 0 bis 10 Gew.-%, wobei die Gesamtzusammensetzung einen 100 Gew.-% beträgt. Als andere Metalloxide und Halbmetalloxide sind bevorzugt Yttrium Oxid, insbesondere Y₂O₃, MgO, Al₂O₃.

Ein besonders bevorzugter Gegenstand der Erfindung ist ein keramischer Rohling umfassend ein keramisches Gerüst, insbesondere ein offenporiges keramisches Gerüst, umfassend einen Gehalt an Zirkondioxid von 50 bis 99,9 Gew.-%, insbesondere von 70 bis 99,9 Gew.-%, bevorzugt von 85 Gew.-% bis 99,9 Gew.-%, besonders bevorzugt von 90 bis 99,9 Gew.-% oder von 50 bis 97 Gew.-%, und optional umfassend einen Gehalt anderer Metalloxide, Halbmetalloxide, Siliciumcarbid, insbesondere eines der vorgenannten, oder Gemischen dieser ausgewählt aus Yttrium, Aluminium, Magnesium, Kalium, Calcium, Lithium, und optional Silicium, mit einem Gehalt von 0,1 bis 50 Gew.-% oder von 3 bis 50 Gew.-%, insbesondere von 0,1 bis 30 Gew.-%, bevorzugt von 0,1 bis 15 Gew.-%, besonders bevorzugt von 0,1 bis 10 Gew.-%, wobei die Gesamtzusammensetzung einen 100 Gew.-% beträgt. Nach weiteren

Alternativen der Erfindung umfasst das keramische Gerüst keinen Feldspat oder Magnesiumaluminat (MgAl₂O₄ Spinell) oder alternativ mit einem Gehalt von 0,001 bis 5 Gew.-%.

Ein erfindungsgemäß besonders bevorzugter keramischer Rohling umfasst ein keramisches Gerüst, insbesondere ein offenporiges keramisches Gerüst, umfassend einen Gehalt an Zirkondioxid von 50 bis 98 Gew.-% oder bis 99,9 Gew.-% vorzugsweise bis 99,99 Gew.-%, insbesondere von 70 bis 98 Gew.-%, vorzugsweise insbesondere von 85 bis 98 Gew.-%, sowie mit einem Gehalt an Yttriumoxid, insbesondere Yttrium(III)oxid, von 0,01 Gew.-% bis 20 Gew.-%, vorzugsweise von 0,1 bis 15 Gew.-%, insbesondere von 1 bis 15 Gew.-%, besonders bevorzugt von 2 bis 15 Gew.-%, sowie optional oder alternativ mit einem Gehalt an Magnesiumoxid von 0,01 bis 10 Gew.-%, insbesondere von 0,1 bis 5 Gew.-%, und/oder optional mit einem Gehalt an Aluminiumoxid, insbesondere Al₂O₃, von 0,01 bis 30 Gew.-%, insbesondere von 0,1 Gew.-% bis 25 Gew.-%, vorzugsweise beträgt der Gesamtgehalt der vorgenannten Komponenten 100 Gew.-%. Typische erfindungsgemäße keramische Gerüste umfassen ZrO₂/Y₂O₃ mit einem Gehalt von 95 Gew.-% an ZrO₂ und 5 Gew.-% Y₂O₃ oder ZrO₂/Y₂O₃/Al₂O₃ mit einem Gehalt von circa 95 Gew.-% ZrO₂ und circa 4 bis 5 Gew.-% Y₂O₃ und circa 0,25 Gew.-% Al₂O₃; ZrO₂/Al₂O₃/Y₂O₃ mit einem Gehalt von circa 76 Gew.-% ZrO₂ und 20 Gew.-% Al₂O₃ und 4 Gew.-% Y₂O₃; ZrO₂/Y₂O₃ mit einem Gehalt von circa 90 Gew.-% ZrO₂ und 10 Gew.-% Y₂O₃; ZrO₂/MgO mit einem Gehalt von circa 96,5 Gew.-% ZrO₂ und 3,5 Gew.-% MgO. Die obigen Gehalte ergänzen sich vorzugsweise immer auf 100 Gew.-% oder optional mit Additiven auf 100 Gew.-%.

Die mindestens eine organische Verbindung umfasst vorzugsweise a) mindestens ein polymerisierbares Monomer und/oder eine Mischung von polymerisierbaren Monomeren, die vorzugsweise flüssige, polymerisierbare Monomere umfasst, und/oder b) Polymere, insbesondere Polymere der vorgenannten Monomere, und/oder c) Wachs, insbesondere ein verflüssigbares Wachs, insbesondere ein unzersetzt verflüssigbares Wachs. Als Wachs kommen beispielsweise dem Fachmann bekannte Klebewachse, Fräs- und Universalwachse in Frage, wie insbesondere Paraffin enthaltende Wachse.

Der erfindungsgemäße Rohling kann dann vorzugsweise mit deutlich geringerem Materialausschuss in einem automatisierten Fräsverfahren verwendet werden, da die Bruchfestigkeit bzw. Biegefestigkeit und der E-Modul die Rohlinge deutlich besser die fräsende Bearbeitung ohne ein Abplatzen von Teilen des Rohlings während des Fräsvorgangs überstehen. Ferner kann häufiger auf die Verwendung von Stegen zwischen Formteil und Restrohling, die einen Verzug der Formteile beim anschließenden Sintervorgang verhindern sollen, in den erfindungs-gemäßen Rohlingen verzichtet werden. Das Sintern der Formteile erfolgt in der Regel bei Temperaturen von 1000 bis 1500 °C.

Besonders bevorzugt umfasst das mindestens eine Monomer 2-Hydroxymethacrylt oder eine Mischung mit 2-Hydroxymethacrylt. Weitere ebenfalls bevorzugte Monomere umfassen a) mindestens ein polymerisierbares Monomer und/oder Mischungen von polymerisierbaren Monomeren, wobei das Monomer ausgewählt ist aus monofunktionellen Monomeren umfassend 2-Hydroxyethylmethacrylat (HEMA, Glykolmethylcrylat), Alkylmethacrylate, (Methyl)methacrylat und/oder mindestens einem di-, tri-, tetra- oder multi-funktionelles Monomer 1,4-Butandiol-dimethacrylat (1,4-BDMA) oder Pentaerythritol-tetraacrylat, Bis-GMA-Monomer (Bisphenyl-A-Glycidyl-Methacrylat), Triethylen-glycoldimethacrylat (TEGDMA) und Diethylenglycoldimethacrylat (DEGMA), Tetraethylen-glykoldi(meth)acrylat, propoxyliertes Neopentylglykoldiacrylate, Alkyldioldi(meth)acrylat mit C2 bis C15 in der Alkyl-Gruppe, Decandioldi(meth)acrylat, Dodecandioldi(meth)acrylat, Hexyldecan-dioldi(meth)acrylat, Trimethylolpropantri(meth)acrylat, Pentaerythrittetra(meth)-acrylat sowie Butandioldi(meth)-acrylat, Ethylenglycol-di(meth)acrylat, Polyethylenglycol-di(meth)acrylate, ethoxylierte/ propoxylierte Bisphenol-A-di(meth)acrylate, Tris-(2-Hydroxyethyl)-isocynaurat-Triacrylat, Urethan(meth)acrylat, wie Bis(methacryloxy-2-ethoxycarbonylamino)-alkylen, mit Alkylen von 2 bis 15 C-Atomen, UDMA, eine Mischung enthaltend mindestens eines dieser (Meth-)acrylate und/oder Polymere und/oder Co-Polymere umfassend eines oder mindestens zwei der vorgenannten Monomere. Als geeignete Alkylmethacrylate seien genannt Methyl-, Ethyl-, n-Propyl, i-Propyl-, n-Butyl, t-Butyl, i-Butyl, Benzyl- und Furfurylmethacrylate oder deren Mischungen. Methylmethacrylat ist davon besonders bevorzugt.

Besonders bevorzugte Polymere als die mindestens eine organische Verbindung sind erhältlich durch Polymerisation mindestens eines der vorgenannten Monomere oder einer Mischung umfassend mindestens zwei der vorgenannten Monomere.

Des Weiteren können die Monomere mit thermischen Initiatoren, wie Peroxiden oder Azoverbindungen, versetzt sein.

Das Wachs kann vorzugsweise ausgewählt sein aus dentalen Wachsen, insbesondere umfassend Paraffin, Zeresin, Karnaubawachs, Kakaobutter, Bienenwachs, Stearinsäure und/oder mikrokristalline, paraffinische Kohlenwasserstoffwachsen.

Das Wachs weist eine Schmelztemperatur von größer gleich 140 °C auf, insbesondere größer gleich 145 °C. Diese hochschmelzenden Wachse sind besonders gut für die Infiltration von Fräsrohlingen geeignet. Alternativ kann das Wachs eine Schmelztemperatur von größer 50 °C bis 100°C oder von größer 100° bis 139 °C aufweisen.

Der erfindungsgemäße Rohling ist vorzugsweise ein Weißling, ein Gründling oder ein Rohling im heiß-isostatisch-gepressten Zustand (HIP), vorzugsweise ist der Rohling ein Weißling. Ferner weist der erfindungsgemäße Rohling vorzugsweise eine Bruchfestigkeit bzw. Biegefestigkeit nach der EN DIN 6872 : 2008 größer gleich 41 N/mm² und/oder ein E-Modul von größer gleich 37 kN/mm² auf, gemessen nach 4-Punkt Biegeversuch DIN EN ISO 6872:2008. Des Weiteren weist ein Rohling mit mindestens einem Polymer als organische Verbindung eine Bruchfestigkeit größer gleich 60 N/mm² auf, insbesondere größer gleich 70 N/mm², größer gleich 80 N/mm², größer gleich 90 N/mm², größer gleich 95 N/mm², insbesondere größer gleich 100 N/mm², und optional bis 110 N/mm² auf und/oder ein E-Modul von größer gleich 37 kN/mm² auf, insbesondere größer gleich 40 kN/mm², bevorzugt größer gleich 45 kN/mm², besonders bevorzugt größer gleich 50 kN/mm², vorzugsweise größer gleich 55 kN/mm², weiter bevorzugt von größer gleich 60 kN/mm² gemessen nach 4-Punkt Biegeversuch DIN EN ISO 6872:2008. Besonders bevorzugt sind Rohlinge mit einer Bruchfestigkeit bzw. Biegefestigkeit größer gleich 60 N/mm², insbesondere größer gleich 70 N/mm² auf, vorzugsweise größer gleich 80 N/mm², größer gleich 90 N/mm², insbesondere größer gleich 100 N/mm² mit einem E-Modul von größer gleich 40 kN/mm², bevorzugt größer gleich 50 kN/mm² gemessen nach 4-Punkt Biegeversuch DIN EN ISO 6872:2008.

Ein erfindungsgemäßer Rohling ist besonders gut geeignet zur Herstellung mindestens eines prothetischen dentalen oder medizinischen Rohlings eines Formteils, wie eines Weißlings, in einem materialabtragenden Verfahren, insbesondere einem CAD/CAM-Verfahren, umfassend eine fräsende, bohrende und/oder schneidende Bearbeitung und/oder eine materialabtragende Bearbeitung mittels Laser. Folglich sind Gegenstand der Erfindung Fräsrohlinge und Rohlinge für eine materialabtragende Bearbeitung mittels Laser. Die Weißlinge dentaler Formteile werden anschließend gesintert.

Ebenso Gegenstand der Erfindung ist ein Verfahren zur Herstellung eines Rohlings sowie ein Rohling erhältlich nach dem Verfahren, umfassend mindestens eine organische Verbindung, indem (i) ein Rohling mit einem offenporigen keramischen Gerüst mit mindestens einer flüssigen oder aus der Gasphase abscheidbaren organischen Verbindung in Kontakt gebracht wird, und (ii) das offenporige keramische Gerüst des Rohlings in Bezug auf die Gesamtzusammensetzung des dentalen keramischen Rohlings zu 5 bis 50 Gew.-% in Bezug auf die Gesamtzusammensetzung mindestens eine organische Verbindung aufnimmt.

Dabei ist es bevorzugt, wenn der Rohling mit mindestens einer flüssigen organischen Verbindung infiltriert wird oder mindestens eine aus der Gasphase abscheidbare organische Verbindung in dem Rohling kondensiert wird.

Der Verfahrensschritt (i), in dem ein Rohling mit einem offenporigen keramischen Gerüst mit mindestens einer flüssigen oder aus der Gasphase abscheidbaren organischen Verbindung in Kontakt gebracht wird, kann erfolgen, indem eine Infiltration unter Druck von 1,1 bis 10 kbar, vorzugsweise von 2 bis 10 bar erfolgt oder alternativ wird das offenporige Gerüst zuvor evakuiert, insbesondere indem das offenporige Gerüst im Vakuum bei 10⁻⁵ bar bis 0,9 bar, insbesondere 10⁻³ bar bis 0,1 bar, mit mindestens einer flüssigen oder aus der Gasphase abscheidbaren organischen Verbindung in Kontakt gebracht wird.

Ferner ist es bevorzugt, wenn das offenporige keramische Gerüst des Rohlings eine offenporige Porosität von 10 bis 80 % aufweist, insbesondere eine offenporige Porosität von 20 bis 70 %, bevorzugt von 20 bis 50%, alternativ eine Porosität von ca. 25% +/- 5 %.

Die vorgenannte organische Verbindung umfasst das vorgenannte a) mindestens eine polymerisierbare Monomer und/oder Mischungen von polymerisierbaren Monomeren, und/oder b) Polymere, insbesondere der Monomere aus a), und/oder c) mindestens ein Wachs. Erfindungsgemäß ist es dabei bevorzugt, wenn die organische Verbindung mindestens ein Monomer umfasst und (ii) polymerisiert wird oder die organische Verbindung ein flüssiges Wachs umfasst, das (iii) verfestigt wird, insbesondere abgekühlt wird.

Gleichfalls Gegenstand der Erfindung ist die Verwendung eines flüssigen, polymerisierbaren Monomers, einer Mischung von polymerisierbaren Monomeren und/oder eines Wachses, wie vorstehend aufgeführt, zur Infiltration einer offenporigen dentalen Keramik, insbesondere eines offenporigen keramischen Gerüsts des Rohlings, insbesondere zur Herstellung eines mit mindestens einer organischen Verbindung gefüllten Rohlings.

Als Initiatoren eigenen sich a) Peroxid und/oder Azoverbindung, insbesondere LPO: Dilauroylperoxid, BPO: Dibenzoylperoxid, t-BPEH: tert.-Butylper-2-ethylhexanoat, AIBN: 2,2'-Azobis-(isobutyronitril), DTBP: Di-tert.-Butylperoxid, und optional b) mindestens ein Aktivator, insbesondere mindestens ein aromatisches Amin, wie N,N-Dimethyl-p-toluidin, N,N-Dihydroxyethyl-p-toluidin und/oder p-Dibenzylaminobenzoesäurediethylester oder mindestens ein Initiatorsystem ausgewählt aus Redoxsystemen, insbesondere eine Kombination ausgewählt aus Dibenzoylperoxid, Dilauroylperoxid und Campherchinon mit Aminen ausgewählt aus N,N-Dimethyl-p-toluidin, N-N-Dihydroxyethyl-p-toluidin und p-Dimethylaminobenzoesäurediethylester oder ein Redoxsystem umfassend ein Peroxid, und ein Reduktionsmittel ausgewählt aus Ascorbinsäure, Ascorbinsäurederivat, Barbitursäure oder ein Barbitursäurederivat, Sulfinsäure, Sulfinsäurederviat, besonders bevorzugt ist ein Redoxsystem umfassend
(i) Barbitursäure oder Thiobarbitursäure oder ein Barbitursäure- oder Thiobarbitursäurederivat
und (ii) mindestens ein Kupfersalz oder Kupferkomplex und
(iii) mindestens eine Verbindung mit einem ionischen Halogenatom, besonders bevorzugt ist ein Redoxsystem umfassend 1-Benzyl-5-Phenylbarbitursäure, Kupferacetylacetonat und Benzyldibutylammoniumchlorid. Besonders bevorzugt wird die Polymerisation im 2-Komponenten Dentalmaterial über ein Barbitursäurederivat gestartet.

Im Folgenden werden Ausführungsbeispiele der Erfindung anhand von schematisch dargestellten Figuren und Ausführungsbeispielen erläutert, ohne jedoch dabei die Erfindung zu beschränken. Dabei zeigt:

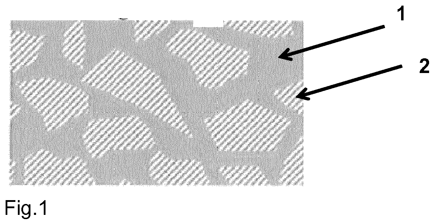

- Figur 1:: Schematisch ein offenporiges keramisches Gerüst 2, das mit einer organischen Verbindung 1 gefüllt ist. Beim Sintern bei 1000 bis 1500 °C wird die organische Verbindung pyrolysiert, so dass das offenporige keramische Gerüst im Wesentlichen ohne organische Verbindung zurückbleibt.

Zirkonoxid im Weisslingszustand (offene Porosität) wurde mit einer aushärtbaren Flüssigkeit infiltriert. Dazu wurden unterschiedliche Infiltrationsmaterialien getestet, wobei Monomer HEMA zu 99,5 Gew.-% und Interox TBPEH zu 0,5 % (flüssiges Peroxid) eingesetzt wurden. Mit diesem Monomer konnte die größte Festigkeitssteigerung erzielt werden, wie der 4-Punkt-Biegeversuch nach DIN EN ISO 6872:2008 belegt. Es wurde ein Zirkonoxid Rohling mit 100 mm Durchmesser und einer Höhe von 14 mm mit HEMA infiltriert. Die Infiltration des Rohlings erfolgt in einem Bad der jeweiligen flüssigen oder organischen Verbindung bis zur vollständigen Sättigung des offenporigen keramischen Gerüstes. Der Rohling wurde in das Monomer gelegt und anschließend bei 90°C ausgehärtet.
Infiltrationszeit:2h; Aushärtezeit bei 90°C:3h

### Gewichtsunterschied:

| Rohling | Weisslingszustand | Infiltrierter Weisslingszustand |
|---|---|---|
| 1 | 342,010g | 387,610g |
| 2 | 341,776g | 380,559g |

**Biegefestigkeiten** nach der EN DIN 6872 : 2008:

### Beispiel 1:

ZrO₂ transluzent weiß, gefräste Proben
Ungesintert, infiltriert rosa

**Tabelle 1a:**

| Nr | Probendicke mm | Proben breite mm | F max N | Bruchfestigkeit N/mm² | E-Mod kN/mm² |
|---|---|---|---|---|---|
| 1 | 3,001 | 4,004 | 117,8 | 73,53 | 43 |
| 2 | 3,006 | 4,002 | 145,8 | 90,69 | 60 |
| 3 | 3,006 | 4,003 | 127,0 | 78,98 | 56 |
| 4 | 3,006 | 4,002 | 141,1 | 87,73 | 56 |
| 5 | 3,005 | 4,004 | 143,3 | 89,09 | 57 |
| 6 | 3,000 | 4,004 | 123,1 | 76,58 | 52 |
| 7 | 3,006 | 4,005 | 114,4 | 71,14 | 54 |
| 8 | 3,006 | 4,005 | 132,7 | 82,48 | 52 |
| 9 | 3,006 | 4,005 | 134,9 | 83,87 | 52 |
| 10 | 3,005 | 4,006 | 162,3 | 100,85 | 62 |
| 11 | 3,005 | 4,006 | 133,9 | 83,26 | 54 |
| 12 | 3,006 | 4,004 | 146,8 | 91,28 | 56 |
| 13 | 3,005 | 4,003 | 111,0 | 69,06 | 51 |
| 14 | 3,005 | 4,004 | 169,2 | 105,28 | 40 |

**Tabelle 1b:**

| Serie n = 14 | F max N | Bruchfestigkeit N/mm² | E-Mod kN/mm² |
|---|---|---|---|
| X | 135,9 | 84,56 | 53 |

### Beispiel 2:

ZrO₂ transluzent weiß, gefräste Proben.
Ungesintert, infiltriert weiß

**Tabelle 2a:**

| Nr | Probendicke mm | Probenbreite | F max N | Bruchfestigkeit | E-Mod |
|---|---|---|---|---|---|
| | | mm | | N/mm² | kN/mm² |
| 1 | 3,006 | 4,005 | 153,3 | 95,33 | 62 |
| 2 | 3,002 | 4,003 | 165,0 | 102,89 | 59 |
| 3 | 3,006 | 4,005 | 164,6 | 102,35 | 56 |
| 4 | 3,005 | 4,005 | 167,5 | 104,24 | 60 |
| 5 | 3,006 | 4,004 | 145,2 | 90,27 | 58 |
| 6 | 3,006 | 4,004 | 163,5 | 101,68 | 58 |
| 7 | 3,006 | 4,006 | 145,8 | 90,60 | 59 |
| 8 | 3,000 | 4,006 | 171,3 | 106,43 | 58 |
| 9 | 3,006 | 4,006 | 125,8 | 78,17 | 60 |
| 10 | 3,005 | 4,005 | 138,3 | 86,02 | 59 |
| 11 | 3,007 | 4,003 | 159,5 | 99,17 | 59 |
| 12 | 3,003 | 4,005 | 133,2 | 82,97 | 58 |
| 13 | 3,003 | 4,005 | 124,6 | 77,60 | 55 |
| 14 | 3,005 | 4,005 | 161,4 | 100,43 | 54 |

**Tabelle 2b:**

| Serie n = 14 | F max N | Bruchfestigkeit N/mm² | E-Mod kN/mm² |
|---|---|---|---|
| X | 151,4 | 94,15 | 58 |

### Beispiel 3:

ZrO₂ transluzent weiß, gefräste Proben.
Ungesintert, unbearbeitet, Wachs

**Tabelle 3a:**

| Nr | Probendicke mm | Proben breite mm | F max N | Bruchfestigkeit N/mm² | E-Mod kN/mm² |
|---|---|---|---|---|---|
| 1 | 3,026 | 4,068 | 76,8 | 46,42 | 37 |
| 2 | 3,019 | 4,070 | 71,9 | 43,58 | 39 |
| 3 | 3,028 | 4,088 | 74,5 | 44,73 | 39 |
| 4 | 3,026 | 4,061 | 74,2 | 44,88 | 40 |
| 5 | 3,023 | 4,063 | 67,7 | 41,03 | 40 |
| 6 | 3,025 | 4,091 | 81,7 | 49,09 | 40 |
| 7 | 3,040 | 4,063 | 81,9 | 49,09 | 40 |
| 8 | 3,012 | 4,065 | 78,8 | 47,59 | 37 |
| 9 | 3,016 | 4,078 | 72,0 | 43,69 | 38 |
| 10 | 3,021 | 4,080 | 67,9 | 41,02 | 37 |
| 11 | 3,027 | 4,083 | 70,9 | 42,64 | 37 |
| 12 | 3,025 | 4,067 | 79,8 | 48,23 | 35 |

**Tabelle 3b:**

| Serie n = 12 | F max N | Bruchfestigkeit N/mm² | E-Mod kN/mm² |
|---|---|---|---|
| X | 74,8 | 45,17 | 38 |

### Beispiel 4: Vergleichsbeispiel (nicht infiltriert)

ZrO₂ transluzent weiß, gefräste Proben.
Ungesintert, unbearbeitet.

**Tabelle 4a:**

| | Probendicke | Proben breite | F max | Bruchfestigkeit | E-Mod |
|---|---|---|---|---|---|
| Nr | mm | mm | N | N/mm² | kN/mm² |
| 1 | 3,018 | 4,068 | 64,4 | 39,13 | 36 |
| 2 | 3,020 | 4,068 | 67,9 | 41,17 | 35 |
| 3 | 3,021 | 4,077 | 67,2 | 40,64 | 35 |
| 4 | 3,023 | 4,070 | 62,9 | 38,05 | 36 |
| 5 | 3,006 | 4,081 | 66,9 | 40,79 | 36 |
| 6 | 3,019 | 4,062 | 66,3 | 40,26 | 35 |
| 7 | 3,024 | 4,062 | 63,5 | 38,46 | 35 |
| 8 | 3,022 | 4,065 | 69,7 | 42,24 | 35 |
| 9 | 3,014 | 4,069 | 65,6 | 39,96 | 36 |
| 10 | 3,023 | 4,067 | 69,4 | 42,04 | 35 |
| 11 | 3,020 | 4,067 | 67,3 | 40,88 | 35 |
| 12 | 3,015 | 4,067 | 62,4 | 37,96 | 35 |
| 13 | 3,018 | 4,072 | 66,8 | 40,50 | 35 |
| 14 | 3,024 | 4,060 | 67,7 | 41,04 | 35 |

**Tabelle 4b:**

| Serie n = 14 | F max N | Bruchfestigkeit N/mm² | E-Mod kN/mm² |
|---|---|---|---|
| X | 66,3 | 40,22 | 35 |

## Patentansprüche

1. Dentaler keramischer Rohling umfassend mindestens eine organische Verbindung,
wobei der Rohling teilgesintert ist und ein offenporiges keramisches Gerüst aufweist, **dadurch gekennzeichnet, dass**
das offenporige keramische Gerüst ausgewählt ist aus einem offenporigen dentalen keramischen Gerüst umfassend Zirkondioxid, Aluminiumoxid, Mischoxid umfassend Zirkondioxid, und/oder Siliciumcarbid, wobei das offenporige keramische Gerüst von 5 bis 50 Gew.-% mindestens eine organische Verbindung in Bezug auf die Gesamtzusammensetzung des dentalen keramischen Rohlings aufweist, wobei mindestens eine organische Verbindung ausgewählt ist aus
a) mindestens einem polymerisierbaren Monomer und/oder Mischung von polymerisierbaren Monomeren, wobei das Monomer ausgewählt ist aus mono-funktionellen Monomeren umfassend 2-Hydroxyethylmethacrylat (HEMA), Glykolmethylacrylat, Alkylmethacrylate, (Methyl)methacrylat und/oder mindestens einem di-, tri-, tetra- oder multi-funktionellen Monomer umfassend 1,4-Butandioldimethacrylat (1,4-BDMA) oder Pentaerythritoltetraacrylat, Bis-GMA-Monomer (Bisphenyl-A-Glycidyl-Methacrylat), Triethylenglycoldimethacrylat (TEGDMA) und Diethylenglycoldimethacrylat (DEGMA), Tetraethylenglykoldi(meth)acrylat, propoxyliertes Neopentylglykoldiacrylate, Alkyldioldi(meth)acrylat mit C2 bis C15 in der Alkyl-Gruppe, Decandioldi(meth)acrylat, Dodecandioldi(meth)acrylat, Hexyldecan-dioldi(meth)acrylat, Trimethylolpropantri(meth)acrylat, Pentaerythritoltetra(meth)acrylat sowie Butandioldi(meth)acrylat, Ethylenglycoldi(meth)acrylat, Polyethylenglycol-di(meth)acrylate, ethoxylierte/propoxylierte Bisphenol-A-di(meth)acrylate, Tris-(2-Hydroxyethyl)isocyanurat-Triacrylat, Urethan(meth)acrylat, wie Bis(methacryloxy-2-ethoxycarbonylamino)alkylen, mit Alkylen von 2 bis 15 C-Atomen, UDMA, eine Mischung enthaltend mindestens eines dieser (Meth)acrylate,
b) Polymeren erhältlich durch Polymerisation mindestens eines der vorgenannten Monomere oder einer Mischung umfassend mindestens zwei der vorgenannten Monomere, und/oder
c) Wachs mit einer Schmelztemperatur größer gleich 140 °C, wobei das Wachs dentale Wachse umfasst, umfassend Paraffin, Zeresin, Karnaubawachs, Kakaobutter, Bienenwachs, Stearinsäure und/oder mikrokristalline, paraffinische Kohlenwasserstoffwachse.

2. Rohling nach Anspruch 1, **dadurch gekennzeichnet, dass**
der Rohling zu 5 bis 25 Gew.-% in Bezug auf die Gesamtzusammensetzung mindestens eine organische Verbindung enthält.

3. Rohling nach Anspruch 1 oder 2, **dadurch gekennzeichnet, dass**
der Rohling ein Weißling, ein Grünling oder ein Rohling im heiß-isostatisch-gepressten Zustand (HIP) ist, vorzugsweise ist der Rohling ein Weißling.

4. Rohling nach einem der Ansprüche 1 bis 3, **dadurch gekennzeichnet, dass**
der Rohling eine Bruchfestigkeit größer gleich 41 N/mm² und/oder ein E-Modul von größer gleich 37 kN/mm² aufweist gemessen nach 4-Punkt Biegeversuch DIN EN ISO 6872:2008.

5. Rohling nach einem der Ansprüche 1 bis 4, **dadurch gekennzeichnet, dass**
der Rohling geeignet ist zur Herstellung mindestens eines prothetischen dentalen oder medizinischen Rohlings eines Formteils in einem materialabtragenden Verfahren, insbesondere einem CAD/CAM-Verfahren, umfassend eine fräsende, bohrende und/oder schneidende Bearbeitung und/oder eine materialabtragende Bearbeitung mittels Laser.

6. Rohling nach einem der Ansprüche 1 bis 5, **dadurch gekennzeichnet, dass**
das offenporige keramische Gerüst eine offenporige Porosität von 10 bis 80 % aufweist, insbesondere eine offenporige Porosität von 20 bis 70 %, bevorzugt von 20 bis 60%.

7. Verfahren zur Herstellung eines Rohlings umfassend mindestens eine organische Verbindung nach einem der Ansprüche 1 bis 6, indem
(i) ein teilgesinterter Rohling mit einem offenporigen keramischen Gerüst das ausgewählt ist aus einem offenporigen dentalen keramischen Gerüst umfassend Zirkondioxid, Aluminiumoxid, Mischoxid umfassend Zirkondioxid, und/oder Siliciumcarbid, mit mindestens einer flüssigen oder aus der Gasphase abscheidbaren organischen Verbindung in Kontakt gebracht wird, wobei der Rohling mit mindestens einer flüssigen organischen Verbindung infiltriert wird oder mindestens eine aus der Gasphase abscheidbare organische Verbindung in dem Rohling kondensiert wird,
wobei mindestens eine organische Verbindung ausgewählt ist aus
a) mindestens einem polymerisierbaren Monomer und/oder Mischung von polymerisierbaren Monomeren, wobei das Monomer ausgewählt ist aus mono-funktionellen Monomeren umfassend 2-Hydroxyethylmethacrylat (HEMA), Glykolmethylacrylat, Alkylmethacrylate, (Methyl)methacrylat und/oder mindestens einem di-, tri-, tetra- oder multi-funktionellen Monomer umfassend 1,4-Butandioldimethacrylat (1,4-BDMA) oder Pentaerythritoltetraacrylat, Bis-GMA-Monomer (Bisphenyl-A-Glycidyl-Methacrylat), Triethylenglycoldimethacrylat (TEGDMA) und Diethylenglycoldimethacrylat (DEGMA), Tetraethylenglykoldi(meth)acrylat, propoxyliertes Neopentylglykoldiacrylate, Alkyldioldi(meth)acrylat mit C2 bis C15 in der Alkyl-Gruppe, Decandioldi(meth)acrylat, Dodecandioldi(meth)acrylat, Hexyldecan-dioldi(meth)acrylat, Trimethylolpropantri(meth)acrylat, Pentaerythritoltetra(meth)acrylat sowie Butandioldi(meth)acrylat, Ethylenglycoldi(meth)acrylat, Polyethylenglycol-di(meth)acrylate, ethoxylierte/ propoxylierte Bisphenol-A-di(meth)acrylate, Tris-(2-Hydroxyethyl)isocyanurat-Triacrylat, Urethan(meth)acrylat, wie Bis(methacryloxy-2-ethoxycarbonylamino)alkylen, mit Alkylen von 2 bis 15 C-Atomen, UDMA, eine Mischung enthaltend mindestens eines dieser (Meth)acrylate, eine Mischung enthaltend mindestens eines dieser (Meth)acrylate und/oder Polymere und/oder Co-Polymere umfassend eines oder mindestens zwei der vorgenannten Monomere,
und/oder
b) Wachs mit einer Schmelztemperatur größer gleich 140 °C, wobei das Wachs dentale Wachse umfasst, umfassend Paraffin, Zeresin, Karnaubawachs, Kakaobutter, Bienenwachs, Stearinsäure und/oder mikrokristalline, paraffinische Kohlenwasserstoffwachse, und
(ii) das offenporige keramische Gerüst des teilgesinterten Rohlings in Bezug auf die Gesamtzusammensetzung des dentalen keramischen Rohlings zu 5 bis 50 Gew.-% mindestens eine organische Verbindung aufnimmt.

8. Verfahren nach Anspruch 7, **dadurch gekennzeichnet, dass** das offenporige keramische Gerüst des Rohlings eine offenporige Porosität von 10 bis 80 % aufweist und mit mindestens einer flüssigen organischen Verbindung infiltriert wird oder mindestens eine aus der Gasphase abscheidbare organische Verbindung in dem Rohling kondensiert wird.

9. Verfahren nach Anspruch 7 oder 8, **dadurch gekennzeichnet, dass**
(iii) die organische Verbindung polymerisiert wird oder
(iii) das Wachs verfestigt wird.

10. Rohling erhältlich nach einem Verfahren nach einem der Ansprüche 7 bis 9.

11. Verwendung eines flüssigen, polymerisierbaren Monomers, einer Mischung von polymerisierbaren Monomeren und/oder Wachs umfassend
a) mindestens ein polymerisierbares Monomer und/oder Mischung von polymerisierbaren Monomeren, wobei das Monomer ausgewählt ist aus mono-funktionellen Monomeren umfassend 2-Hydroxyethylmethacrylat (HEMA), Glykolmethylacrylat, Alkylmethacrylate, (Methyl)methacrylat und/oder mindestens einem di-, tri-, tetra- oder multi-funktionelles Monomer umfassend 1,4-Butandioldimethacrylat (1,4-BDMA) oder Pentaerythritoltetraacrylat, Bis-GMA-Monomer (Bisphenyl-A-Glycidyl-Methacrylat), Triethylenglycoldimethacrylat (TEGDMA) und Diethylenglycoldimethacrylat (DEGMA), Tetraethylen-glykoldi(meth)acrylat, propoxyliertes Neopentylglykoldiacrylate, Alkyldioldi(meth)acrylat mit C2 bis C15 in der Alkyl-Gruppe, Decandioldi(meth)acrylat, Dodecandioldi(meth)acrylat, Hexyldecandioldi(meth)acrylat, Trimethylolpropantri (meth)acrylat, Pentaerythritoltetra(meth)acrylat sowie Butandioldi(meth)acrylat, Ethylenglycoldi(meth)acrylat, Polyethylenglycoldi(meth)acrylate, ethoxylierte/ propoxylierte Bisphenol-A-di(meth)acrylate, Tris-(2-Hydroxyethyl)isocyanurat-Triacrylat, Urethan(meth)acrylat, wie Bis(methacryloxy-2-ethoxycarbonylamino)alkylen, mit Alkylen von 2 bis 15 C-Atomen, UDMA, eine Mischung enthaltend mindestens eines dieser (Meth)acrylate und/oder
b) Polymere erhältlich durch Polymerisation mindestens eines der vorgenannten Monomere oder einer Mischung umfassend mindestens zwei der vorgenannten Monomere, und/oder
c) Wachs mit einer Schmelztemperatur größer gleich 140 °C, wobei das Wachs dentale Wachse umfasst, umfassend Paraffin, Zeresin, Karnaubawachs, Kakaobutter, Bienenwachs, Stearinsäure und/oder mikrokristalline, paraffinische Kohlenwasserstoffwachse,
zur Infiltration eines offenporigen keramischen Gerüsts eines teilgesinterten Rohlings mit 5 bis 50 Gew.-% mindestens eine organischen Verbindung in Bezug auf die Gesamtzusammensetzung des dentalen keramischen Rohlings, wobei das offenporige keramische Gerüst ausgewählt ist aus einem offenporigen dentalen keramischen Gerüst umfassend Zirkondioxid, Aluminiumoxid, Mischoxid umfassend Zirkondioxid, und/oder Siliciumcarbid.

## Claims

1. Dental ceramic blank comprising at least one organic compound,
the blank being partially sintered and having an open-pored ceramic scaffold,
**characterised in that**
the open-pored ceramic scaffold is selected from an open-pored dental ceramic scaffold comprising zirconium dioxide, aluminium oxide, mixed oxide comprising zirconium dioxide and/or silicon carbide, the open-pored ceramic scaffold having from 5 to 50 % by weight at least one organic compound, based on the total composition of the dental ceramic blank, the at least one organic compound being selected from
a) at least one polymerisable monomer and/or mixture of polymerisable monomers, the monomer being selected from
mono-functional monomers comprising 2-hydroxyethyl methacrylate (HEMA), glycol methyl acrylate, alkyl methacrylate, (methyl) methacrylate and/or
at least one di-, tri-, tetra- or multi-functional monomer comprising 1,4-butanediol dimethacrylate (1,4-BDMA) or pentaerythritol tetraacrylate, bis-GMA monomer (bisphenol-A glycidyl methacrylate), triethylene glycol dimethacrylate (TEGDMA) and diethylene glycol dimethacrylate (DEGMA), tetraethylene glycol di(meth)acrylate, propoxylated neopentyl glycol diacrylate, alkyldiol di(meth)acrylate with C2 to C15 in the alkyl group, decanediol di(meth)acrylate, dodecanediol di(meth)acrylate, hexyldecanediol di(meth)acrylate, trimethylolpropane tri(meth)acrylate, pentaerythritol tetra(meth)acrylate as well as butanediol di(meth)acrylate, ethylene glycol di(meth)acrylate, polyethylene glycol di(meth)acrylates, ethoxylated/propoxylated bisphenol-A di(meth)acrylates, tris(2-hydroxyethyl) isocyanurate triacrylate, urethane (meth)acrylate, such as bis(methacryloxy-2-ethoxycarbonylamino) alkylene, with alkylene from 2 to 15 C-atoms, UDMA, a mixture containing at least one of said (meth)acrylates,
b) polymers obtainable by polymerising at least one of the afore-mentioned monomers or of a mixture comprising at least two of the afore-mentioned monomers, and/or
c) wax having a melting temperature of greater than or equal to 140 °C, the wax comprising dental waxes, comprising paraffin, ceresin, carnauba wax, cacao butter, beeswax, stearic acid and/or microcrystalline paraffinic hydrocarbon waxes.

2. Blank according to claim 1, **characterised in that**
the blank comprises 5 to 25 % by weight at least one organic compound, based on the total composition.

3. Blank according to claim 1 or 2, **characterised in that**
the blank is a white body, green body or a blank in hot-isostatically pressed state (HIP), preferably the blank is a white body.

4. Blank according to any one of the claims 1 to 3, **characterised in that**
the blank has a fracture strength of greater than or equal to 41 N/mm² and/or an E-modulus of greater than or equal to 37 kN/mm², measured by 4-point bending test DIN EN ISO 6872:2008.

5. Blank according to any one of the claims 1 to 4, **characterised in that**
the blank is suitable for producing at least one prosthetic dental or medical blank of a moulded part in a material-removing process, in particular a CAD/CAM-process, comprising a milling, drilling and/or cutting processing and/or a material-removing processing by means of laser.

6. Blank according to any one of the claims 1 to 5, **characterised in that**
the open-pored ceramic scaffold has an open-pored porosity of 10 to 80 %, in particular an open-pored porosity of 20 to 70 %, preferably of 20 to 60 %.

7. Method for producing a blank comprising at least one organic compound according to any one of the claims 1 to 6, in which
(i) a partially sintered blank having an open-pored ceramic scaffold being selected from an open-pored dental ceramic scaffold comprising zirconium dioxide, aluminium oxide, mixed oxide comprising zirconium dioxide and/or silicon carbide is contacted with at least one liquid organic compound or organic compound depositable from gaseous phase, the blank being infiltrated with at least one liquid organic compound or at least one organic compound depositable from gaseous phase being condensed in the blank, at least one organic compound being selected from
a) at least one polymerisable monomer and/or mixture of polymerisable monomers, the monomer being selected from
mono-functional monomers comprising 2-hydroxyethyl methacrylate (HEMA), glycol methyl acrylate, alkyl methacrylate, (methyl) methacrylate and/or
at least one di-, tri-, tetra- or multi-functional monomer comprising 1,4-butanediol dimethacrylate (1,4-BDMA) or pentaerythritol tetraacrylate, bis-GMA monomer (bisphenol-A glycidyl methacrylate), triethylene glycol dimethacrylate (TEGDMA) and diethylene glycol dimethacrylate (DEGMA), tetraethylene glycol di(meth)acrylate, propoxylated neopentyl glycol diacrylate, alkyldiol di(meth)acrylate with C2 to C15 in the alkyl group, decanediol di(meth)acrylate, dodecanediol di(meth)acrylate, hexyldecanediol di(meth)acrylate, trimethylolpropane tri(meth)acrylate, pentaerythritol tetra(meth)acrylate as well as butanediol di(meth)acrylate, ethylene glycol di(meth)acrylate, polyethylene glycol di(meth)acrylates, ethoxylated/propoxylated bisphenol-A di(meth)acrylates, tris(2-hydroxyethyl) isocyanurate triacrylate, urethane (meth)acrylate, such as bis(methacryloxy-2-ethoxycarbonylamino) alkylene, with alkylene from 2 to 15 C-atoms, UDMA, a mixture containing at least one of said (meth)acrylates, a mixture comprising at least one of said (meth)acrylates and/or polymers and/or copolymers comprising one or at least two of the afore-mentioned monomers,
and/or
b) wax having a melting temperature of greater than or equal to 140 °C, the wax comprising dental waxes, comprising paraffin, ceresin, carnauba wax, cacao butter, beeswax, stearic acid and/or microcrystalline paraffinic hydrocarbon waxes, and (ii) the open-pored ceramic scaffold of the partially sintered blank incorporates 5 to 50 % by weight at least one organic compound, based on the total composition of the dental ceramic blank.

8. Method according to claim 7, **characterised in that**
the open-pored ceramic scaffold of the blank has an open-pored porosity of 10 to 80 % and is infiltrated with at least one liquid organic compound or at least one organic compound depositable from gaseous phase is condensed in the blank.

9. Method according to claim 7 or 8, **characterised in that**
(iii) the organic compound is polymerised, or
(iii) the wax is solidified.

10. Blank obtainable according to a method according to any one of the claims 7 to 9.

11. Use of a liquid polymerisable monomer, a mixture of polymerisable monomers and/or wax comprising
a) at least one polymerisable monomer and/or mixture of polymerisable monomers, the monomer being selected from mono-functional monomers comprising 2-hydroxyethyl methacrylate (HEMA), glycol methyl acrylate, alkyl methacrylate, (methyl) methacrylate and/or at least one di-, tri-, tetra- or multi-functional monomer comprising 1,4 butanediol dimethacrylate (1,4-BDMA) or pentaerythritol tetraacrylate, bis-GMA monomer (bisphenol-A glycidyl methacrylate), triethylene glycol dimethacrylate (TEGDMA) and diethylene glycol dimethacrylate (DEGMA), tetraethylene glycol di(meth)acrylate, propoxylated neopentyl glycol diacrylate, alkyldiol di(meth)acrylate with C2 to C15 in the alkyl group, decanediol di(meth)acrylate, dodecanediol di(meth)acrylate, hexyldecanediol di(meth)acrylate, trimethylolpropane tri(meth)acrylate, pentaerythritol tetra(meth)acrylate as well as butanediol di(meth)acrylate, ethylene glycol di(meth)acrylate, polyethylene glycol di(meth)acrylates, ethoxylated/propoxylated bisphenol-A di(meth)acrylates, tris(2-hydroxyethyl) isocyanurate triacrylate, urethane (meth)acrylate, such as bis(methacryloxy-2-ethoxycarbonylamino) alkylene, with alkylene from 2 to 15 C-atoms, UDMA, a mixture containing at least one of said (meth)acrylates, and/or
b) polymers obtainable by polymerising at least one of the afore-mentioned monomers or of a mixture comprising at least two of the afore-mentioned monomers, and/or
c) wax having a melting temperature of greater than or equal to 140 °C, the wax comprising dental waxes, comprising paraffin, ceresin, carnauba wax, cacao butter, beeswax, stearic acid and/or microcrystalline paraffinic hydrocarbon waxes, for infiltrating an open-pored ceramic scaffold of a partially sintered blank having 5 to 50 % by weight at least one organic compound, based on the total composition of the dental ceramic blank, the open-pored ceramic scaffold being selected from an open-pored dental ceramic scaffold comprising zirconium dioxide, aluminium oxide, mixed oxide comprising zirconium dioxide and/or silicon carbide.

## Revendications

1. Ébauche céramique dentaire comprenant au moins un composé organique, l'ébauche étant frittée partiellement et ayant une armature céramique à pores ouverts,
**caractérisée en ce que**
l'armature céramique à pores ouverts est sélectionnée parmi d'une armature céramique dentaire à pores ouverts comprenant le dioxyde de zirconium, l'oxyde d'aluminium, de l'oxide mixte comprenant le dioxyde de zirconium et/ou le carbure de silicium, l'armature céramique à pores ouverts ayant de 5 à 50 % en poids au moins un composé organique, sur la base de la composition totale de l'ébauche céramique dentaire, l'au moins un composé organique étant sélectionné parmi d'
a) au moins un monomère polymérisable et/ou du mélange de monomères polymérisables, le monomère étant sélectionné parmi des monomères mono-fonctionnels comprenant le méthacrylate de 2-hydroxyéthyle (HEMA), l'acrylate de méthyle de glycol, le méthacrylate d'alkyle, le méthacrylate (de méthyl) et/ou
d'au moins un monomère di-, tri-, tetra- ou multi-fonctionnel comprenant le diméthacrylate de 1,4-butanediol (1,4-BDMA) ou le tétraacrylate de pentaérythritol, le monomère de bis-GMA (le méthacrylate de glycidyle de bisphénol-A), le diméthacrylate de glycol de triéthylène (TEGDMA) et le diméthacrylate de glycol de diéthylène (DEGMA), le di(méth)acrylate de glycol de tétraéthylène, le diacrylate de glycol de néopentyle propoxylé, le di(méth)acrylate d'alkylediol avec C2 à C15 dans la group d'alkyle, le di(méth)acrylate de décanediol, le di(méth)acrylate de dodécanediol, le di(méth)acrylate de décanediol de héxyle, le tri(méth)acrylate de triméthylolpropane, le tétra(méth)acrylate de pentaérythritol ainsi que le di(méth)acrylate de butanediol, le di(méth)acrylate de glycol d'éthylène, les di(méth)acrylates de glycol de polyéthylène, le di(méth)acrylates de bisphénol-A ethoxylés/propoxylés, le triacrylate d'isocyanurate de tris(2-hydroxyéthyle), le (méth)acrylate d'uréthane, tel que l'alkylène de bis(méthacryloxy-2-éthoxycarbonylamino), avec l'alkylène de 2 à 15 atomes C, UDMA, un mélange contenant au moins un desdits (méth)acrylates,
b) des polymères obténable par polymériser au moins un des monomères susmentionnés ou d'un mélange comprenant au moins deux des monomères susmentionnés, et/ou
c) de la cire ayant une température de fusion de supérieure ou égale à 140 °C, la cire comprenant des cires dentaires, comprenant la paraffine, la cérésine, la cire de carnauba, le beurre de cacao, la cire d'abeille, l'acide stéarique et/ou des cires d'hydrocarbure parafiniques microcristallines.

2. Ébauche selon la revendication 1, **caractérisée en ce que**
l'ébauche comprend 5 à 25 % en poids au moins un composé organique, sur la base de la composition totale.

3. Ébauche selon la revendication 1 ou 2, **caractérisée en ce que**
l'ébauche est un corps blanc, corps vert ou une ébauche dans l'état pressée isostatique à chaud (HIP), de préférence l'ébauche est un corps blanc.

4. Ébauche selon l'une des revendications 1 à 3, **caractérisée en ce que**
l'ébauche a une résistance à la rupture de supérieure ou égale à 41 N/mm² et/ou un module élastique de supérieur ou égal à 37 kN/mm², mesuré par l'essaie de flexion 4 points DIN EN ISO 6872:2008.

5. Ébauche selon l'une des revendications 1 à 4, **caractérisée en ce que**
l'ébauche est propre à produire au moins une ébauche dentaire ou médicale prothétique d'une pièce moulée dans un procédé enlevant du matière, en particulier un procédé CAO/FAO, comprenant un usinage fraisant, forant et/ou coupant et/ou un usinage enlevant du matière par moyen d'un laser.

6. Ébauche selon l'une des revendications 1 à 5, **caractérisée en ce que**
l'armature céramique à pores ouverts a une porosité à pores ouverts de 10 à 80 %, en particulier une porosité à pores ouverts de 20 à 70 %, de préférence de 20 à 60 %.

7. Procédé pour produire une ébauche comprenant au moins un composé organique selon l'une des revendications 1 à 6, dans lequel
(i) une ébauche frittée partiellement ayant une armature céramiques à pores ouverts étant sélectionnée parmi d'une armature céramique dentaire à pores ouverts comprenant le dioxyde de zirconium, l'oxyde d'aluminium, de l'oxyde mixte comprenant le dioxyde de zirconium et/ou le carbure de silicium est contactée avec au moins un composé organique liquide ou un composé organique déposable de la phase gazeuse, l'ébauche étant infiltrée avec au moins un composé organique liquide ou au moins un composé organique déposable de la phase gazeuse étant condensé dans l'ébauche, au moins un composé organique étant sélectionné parmi de
a) au moins un monomère polymérisable et/ou du mélange de monomères polymérisables, le monomère étant sélectionné parmi des
monomères mono-fonctionnels comprenant le méthacrylate de 2-hydroxyéthyle (HEMA), l'acrylate de méthyle de glycol, le méthacrylate d'alkyle, le méthacrylate (de méthyl) et/ou
d'au moins un monomère di-, tri- ou multi-fonctionnel comprenant le diméthacrylate de 1,4-butanediol (1,4-BDMA) ou le tétraacrylate de pentaérythritol, le monomère de bis-GMA (le méthacrylate de glycidyle de bisphénol-A), le diméthacrylate de glycol de triéthylène (TEGDMA) et le diméthacrylate de glycol de diéthylène (DEGMA), le di(méth)acrylate de glycol de tétraéthylène, le diacrylate de glycol de néopentyle propoxylé, le di(méth)acrylate d'alkylediol avec C2 à C15 dans la group d'alkyle, le di(méth)acrylate de décanediol, le di(méth)acrylate de dodécanediol, le di(méth)acrylate de décanediol de héxyle, le tri(méth)acrylate de triméthylolpropane, le tétra(méth)acrylate de pentaérythritol ainsi que le di(méth)acrylate de butanediol,
le di(méth)acrylate de glycol d'éthylène, les di(méth)acrylates de glycol de polyéthylène, le di(méth)acrylates de bisphénol-A ethoxylés/propoxylés, le triacrylate d'isocyanurate de tris(2-hydroxyéthyle), le (méth)acrylate d'uréthane, tel que l'alkylène de bis(méthacryloxy-2-éthoxycarbonylamino), avec l'alkylène de 2 à 15 atomes C, UDMA, un mélange contenant au moins un desdits (méth)acrylates, un mélange contenant au moins un desdits (méth)acrylates et/ou des polymères et/ou des copolymères comprenant un ou au moins deux des monomères susmentionnés,
b) de la cire ayant une température de fusion de supérieure ou égale à 140 °C, la cire comprenant des cires dentaires, comprenant la paraffine, la cérésine, la cire de carnauba, le beurre de cacao, la cire d'abeille, l'acide stéarique et/ou des cires d'hydrocarbure parafiniques microcristallines, et
(ii) l'armature céramique à pores ouverts de l'ébauche frittée partiellement incorpore 5 à 50 % en poids au moins un composé organique, sur la base de la composition totale de l'ébauche céramique dentaire.

8. Procédé selon la revendication 7, **caractérisé en ce que**
l'armature céramique à pores ouverts de l'ébauche a une porosité à pores ouverts de 10 à 80 % et est infiltrée avec au moins un composé organique liquide ou au moins un composé organique déposable de la phase gazeuse est condensé dans l'ébauche.

9. Procédé selon la revendication 7 ou 8, **caractérisé en ce que**
(iii) le compose organique est polymérisé, ou
(iii) la cire est solidifiée.

10. Ébauche obténable selon un procédé selon l'une des revendications 7 à 9.

11. Utilisation d'un monomère polymérisable liquide, d'un mélange de monomères polymérisables et/ou de la cire comprenant
a) au moins un monomère polymérisable et/ou du mélange de monomères polymérisables, le monomère étant sélectionné parmi des monomères mono-fonctionnels comprenant le méthacrylate de 2-hydroxyéthyle (HEMA), l'acrylate de méthyle de glycol, le méthacrylate d'alkyle, le méthacrylate (de méthyl) et/ou d'au moins un monomère di-, tri- ou multi-fonctionnel comprenant le diméthacrylate de 1,4-butanediol (1,4-BDMA) ou le tétraacrylate de pentaérythritol, le monomère de bis-GMA (le méthacrylate de glycidyle de bisphénol-A), le diméthacrylate de glycol de triéthylène (TEGDMA) et le diméthacrylate de glycol de diéthylène (DEGMA), le di(méth)acrylate de glycol de tétraéthylène, le diacrylate de glycol de néopentyle propoxylé, le di(méth)acrylate d'alkylediol avec C2 à C15 dans la group d'alkyle, le di(méth)acrylate de décanediol, le di(méth)acrylate de dodécanediol, le di(méth)acrylate de décanediol de héxyle, le tri(méth)acrylate de triméthylolpropane, le tétra(méth)acrylate de pentaérythritol ainsi que le di(méth)acrylate de butanediol, le di(méth)acrylate de glycol d'éthylène, les di(méth)acrylates de glycol de polyéthylène, le di(méth)acrylates de bisphénol-A ethoxylés/propoxylés, le triacrylate d'isocyanurate de tris(2-hydroxyéthyle), le (méth)acrylate d'uréthane, tel que l'alkylène de bis(méthacryloxy-2-éthoxycarbonylamino), avec l'alkylène de 2 à 15 atomes C, UDMA, un mélange contenant au moins un desdits (méth)acrylates,
b) des polymères obténable par polymériser au moins un des monomères susmentionnés ou d'un mélange comprenant au moins deux des monomères susmentionnés, et/ou
c) de la cire ayant une température de fusion de supérieure ou égale à 140 °C, la cire comprenant des cires dentaires, comprenant la paraffine, la cérésine, la cire de carnauba, le beurre de cacao, la cire d'abeille, l'acide stéarique et/ou des cires d'hydrocarbure parafiniques microcristallines,
pour infiltrer une armature céramique à pores ouverts d'une ébauche frittée partiellement ayant 5 à 50 % en poids au moins un composé organique, sur la base de la composition totale de l'ébauche céramique dentaire, l'armature céramique à pores ouverts étant sélectionnée d'une armature céramique dentaire à pores ouverts comprenant le dioxyde de zirconium, l'oxyde d'aluminium, de l'oxyde mixte comprenant le dioxyde de zirconium, et/ou le carbure de silicium.
